# EUROPEAN PATENT SPECIFICATION

(11) **EP 4 245 242 B1**
(45) Date of publication and mention of the grant of the patent: **25.03.2026**
(21) Application number: 23161671.5
(22) Date of filing: 14.03.2023
(51) Int. Cl.: A61B 34/10, G16H 20/40, G16H 30/20, G16H 40/67, G16H 50/50, G16H 30/40

(54) **BONE RESECTION SCORING AND PLANNING**
KNOCHENRESEKTIONSBEWERTUNG UND -PLANUNG
NOTATION ET PLANIFICATION DE RÉSECTION OSSEUSE

(30) Priority: 18.03.2022 US 202263321379 P
(43) Date of publication of application: 20.09.2023
(73) Proprietor: Stryker Australia PTY LTD, Artarmon, NSW 1570 (AU)
(72) Inventor: HILL, David Gibson, Heathmont, Victoria, 3135 (AU); WILLIAMSON, Tom, Brunswick, Victoria 3056 (AU)
(74) Representative: Regimbeau

(56) References cited:
- US-A1- 2010 185 202
- US-A1- 2022 031 473
- US-B2- 10 102 309
- US-B2- 10 881 464

## Description

### Field of the invention

The invention relates to computer-implemented methods for selecting a bone resection plan.

### BACKGROUND OF THE INVENTION

Orthopedic surgery encompasses many different procedures often involving the treatment of damaged or diseased bone. Examples of technologies relevant to orthopedic surgery are disclosed in US 2022/031473, US 10102309, US 10881464 and US 2010/185202. In many of these procedures, a bone is resected to remove the damaged or diseased portion which is then replaced with an implant that restores the function of the bone and provides needed structural support. Before a procedure even begins, a surgeon often develops a pre-operative plan which includes a resection plan that specifies each cut to be made to the bone. These resection plans typically only consider the surgeon's experience with the particular tools at hand and bone preservation. Bone preservation is important at least because bone has its own vascular structure that transports vital nutrients to the bone to keep it alive. Removing more bone than is necessary can lead to necrosis or structural failure which in turn may require a follow-up revision procedure that is typically more complicated than the last. While bone preservation is an important factor to consider, other important factors are often ignored such that the implemented resection plan may not be the most optimal plan for the particular patient. Therefore, further developments are desirable.

### BRIEF SUMMARY OF THE INVENTION

According to the invention, there is provided a computer-implemented method of selecting a bone resection plan for implementation in a surgical procedure as defined in appended claim 1. Advantageous embodiments are defined in the corresponding dependent claims. In one aspect of the present disclosure, a computer-implemented method of selecting a bone resection plan for implementation in a surgical procedure includes generating, by one or more processors, a virtual bone model from a medical image taken of a bone of a mammalian subject and applying, by the one or more processors executing a resection planning application, a plurality of resection plans to the virtual bone model. Each of the plurality of resection plans have at least one virtual control boundary representing a resection of specified geometry and define a region of bone to be removed. Each of the plurality of resection plans have at least one complexity factor and at least one benefit factor associated therewith. The at least one complexity factor and the at least one benefit factor are determined by the one or more processors in the background of the resection planning application upon application of each of the resection plans to the virtual bone model. The method also includes determining, by the one or more processors, a complexity score for each of the resection plans based on the at least one complexity factor. The complexity score is determined in the background of the resection planning application. The method also includes determining, by the one or more processors, a benefit score for each of the resection plans based on the at least one benefit factor. The benefit score is determined in the background of the resection planning application. Also included is the selection of an optimal resection plan from the plurality of resection plans based on the complexity and benefit scores of the plurality of resection plans for execution in the surgical procedure.

In a further aspect of the present disclosure, a method for performing a surgical procedure to replace a portion of bone includes generating, by one or more processors, a virtual bone model from a medical image taken of a bone of a mammalian subject and applying, by the one or more processors executing a resection planning application, a plurality of resection plans to the virtual bone model. Each of the plurality of resection plans have at least one virtual control boundary representing a resection of specified geometry and define a region of bone to be removed. Each of the plurality of resection plans have at least one complexity factor and at least one benefit factor associated therewith. The at least one complexity factor and at least one benefit factor are determined by the one or more processors in the background of the resection planning application upon application of each of the resection plans to the virtual bone model. The method also includes determining, by the one or more processors, a complexity score for each of the resection plans based on the at least one complexity factor and determining, by the one or more processors, a benefit score for each of the resection plans based on the at least one benefit factor. The complexity score and benefit score are determined in the background of the resection planning application. The method further includes selecting an optimal resection plan from the plurality of resection plans based on the complexity and benefit scores of the plurality of resection plans for execution in the surgical procedure, generating, by the one or more processors executing a CAD application, a virtual void filling implant model based on the optimal resection plan, executing the optimal resection plan on the mammalian subject to remove a portion of the bone and form a corresponding void, and implanting a void filling implant based on the virtual void filling implant model into the void.

In an even further aspect of the present disclosure, a non-transitory computer-readable medium includes instructions stored thereon, when executed by a processor, cause the processor to perform a method of selecting a bone resection plan for implementation in a surgical procedure. The method includes generating, by one or more processors, a virtual bone model from a medical image taken of a bone of a mammalian subject and applying, by the one or more processors executing a resection planning application, a plurality of resection plans to the virtual bone model. Each of the plurality of resection plans have at least one virtual control boundary representing a resection of specified geometry and define a region of bone to be removed. Each of the plurality of resection plans have at least one complexity factor and at least one benefit factor associated therewith. The at least one complexity factor and the at least one benefit factor are determined by the one or more processors in the background of the resection planning application upon application of each of the resection plans to the virtual bone model. The method also includes determining, by the one or more processors, a complexity score for each of the resection plans based on the at least one complexity factor. The complexity score is determined in the background of the resection planning application. The method also includes determining, by the one or more processors, a benefit score for each of the resection plans based on the at least one benefit factor. The benefit score is determined in the background of the resection planning application. Also included is the selection of an optimal resection plan from the plurality of resection plans based on the complexity and benefit scores of the plurality of resection plans for execution in the surgical procedure.

### BRIEF DESCRIPTION OF THE DRAWINGS

The features, aspects, and advantages of the present invention will become better understood with regard to the following description, appended claims, and accompanying drawings in which:
FIG. 1 is an exemplary system that may be used in accordance with aspects of the disclosure.
FIG. 2 is a diagram of various devices that may be used in connection with the example system
FIG. 3 is a is a schematic of a Resection Planning Application and Scoring Plug-In of the exemplary system of FIG. 1 according to an embodiment of the present disclosure.
FIG. 4 is a schematic of a Resection Planning Application and Scoring Module of the exemplary system of FIG. 1 according to another embodiment of the present disclosure.
FIG. 5 is a schematic of components of the Scoring Plug-In or Module of FIGs. 3 and 4.
FIG. 6 is a flow diagram describing an example of a method that may be used to implement aspects of the disclosure.
FIG. 7 is bone volume table for a plurality of patient cases and resection plans.
FIG. 8 is a schematic of a bone with a fan cut and blind cut made in the bone.
FIG. 9 is a table of complexity scores for a plurality of patient cases and resection plans.
FIG. 10 is a table of benefit scores for the plurality of patient cases and resection plans of FIG. 9.
FIG. 11 is a perspective view of a virtual bone model of a tibia having a tumor and a plurality of resections of a resection plan according to an embodiment of the present disclosure.
FIG. 12 is a perspective view of a virtual bone model of a femur having a tumor and a plurality of resections of a resection plan according to another embodiment of the present disclosure.
FIG. 13 is a perspective view of a virtual bone model of a femur having a tumor and a plurality of resections of a resection plan according to yet another embodiment of the present disclosure.
FIG. 14 is a perspective view of a virtual bone model of a femur having a tumor and a plurality of resections of a resection plan according to still another embodiment of the present disclosure.
FIG. 15A is a heat map applied to a surface of a distal femur illustrating the magnitude of surface landmark variation for an expected registration error distribution.
FIG. 15B is a schematic of a surface of the distal femur of FIG. 15A illustrating the magnitude of the offset at specified landmarks of such surface for the expected registration error distribution.
FIG. 16 is a graph of complexity and benefits scores of a plurality of resection plans for an exemplary patient case according to an embodiment of the present disclosure.
FIG. 17 is a graph of complexity and benefits scores of a plurality of resection plans for another exemplary patient case according to an embodiment of the present disclosure.

### DETAILED DESCRIPTION

The present disclosure describes systems and methods for performing an orthopedic surgery involving the resection and removal of bone to form a void. The particular circumstances of the patient's condition may require a custom resection plan and a custom implant to fill the void and/or replace the resected bone, such as in a joint replacement procedure. In this regard, the resection of the bone can be approached using any one of a variety of different resection plans that each have their own complexities and benefits. Other aspects of the treatment plan that may follow resection planning may also have their own complexities and benefits. Such aspects may include implant design, manufacture, and implantation. The systems and methods of the present disclosure take these complexities and benefits into account to facilitate the selection of the optimal treatment plan for the particular patient and integration of that treatment plan seamlessly into the creation of the void filling/joint replacement implant and execution of the plan.

The following discussion describes these systems and methods in the context of orthopedic surgery to excise a tumor from a bone, such as a femur or tibia, and fill the remaining void with a void filling implant. Although the examples provided herein are described within such context, it should be understood that the systems and methods described can be applied to other contexts. For example, a revision surgery to replace a previously implanted joint implant, such as a total knee implant, may generate a non-uniform bone void at the end of a bone as a result of damage, disease and/or as incidental to the removal of the implant. The systems and methods described herein can be applied to such revision procedures. In this regard, multiple optimized resection plans for resecting bone around the bone void can be evaluated for complexity and benefit in the same or similar manner described below. A void filling implant can then be generated to fit within the shaped bone void, such as in a press-fit manner. Such void filling implant can also be evaluated for complexity and benefit in the same or similar manner described herein.

A resection of bone can be performed using a variety of tools, such as reamers, reciprocating saws, oscillating saws, mills, drills, osteotomes, rotating burrs, and the like. Regardless of the tool, a resection fundamentally cuts the bone to form the desired shape. Therefore, it should be understood that the present disclosure uses the terms "resection" and "cut" interchangeably.

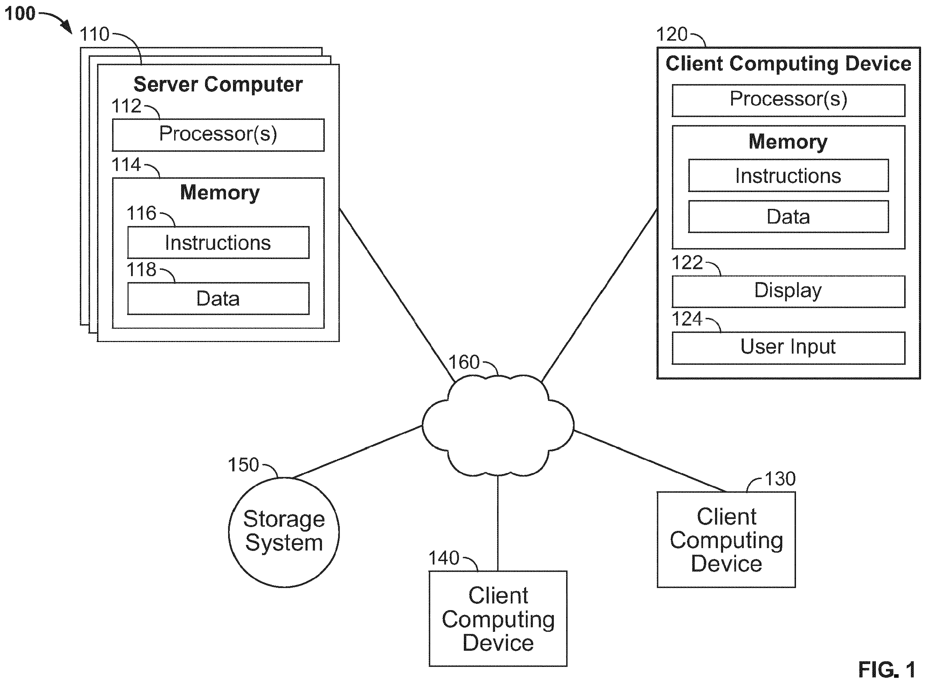

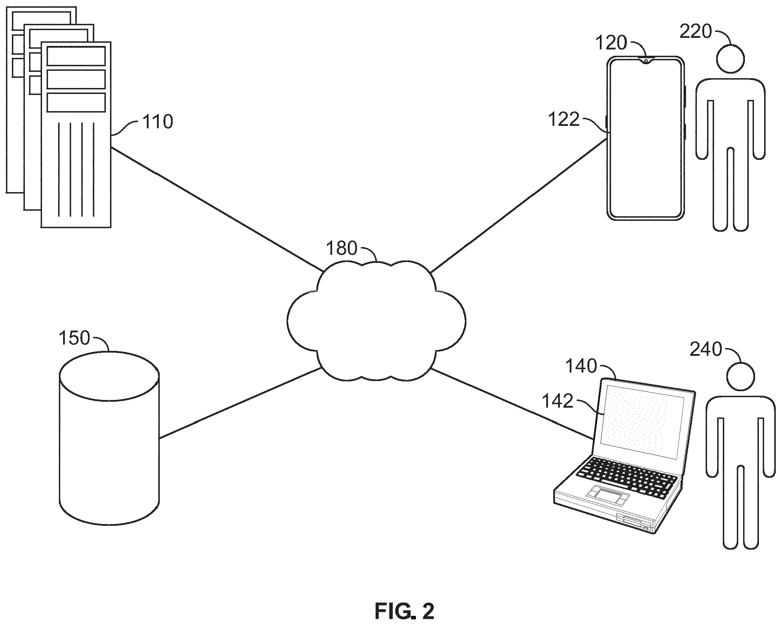

FIGs. 1 and 2 depict an exemplary system 100 for scoring, selecting, and executing a bone resection plan. As an exemplary system, it should not be considered as limiting the scope of the disclosure or usefulness of the features described herein. As shown, system 100 can include computing devices 110, 120, 130, and 140 as well as storage system 150. Each of computing devices 110, 120, 130, and 140 can contain one or more processors 112, memory 114 and other components typically present in general purpose computing devices. Memory 114 of each of computing devices 110, 120, 130, and 140 can store information accessible by the one or more processors 112, including instructions 116 that can be executed by the one or more processors 112.

Memory can also include data 118 that can be retrieved, manipulated, or stored by the processor. The memory can be of any non-transitory type capable of storing information accessible by the processor, such as a hard-drive, memory card, ROM, RAM, DVD, CD-ROM, write-capable, and read-only memories.

The instructions 116 can be any set of instructions to be executed directly, such as machine code, or indirectly, such as scripts, by the one or more processors. In that regard, the terms "instructions," "application," "steps," and "programs" can be used interchangeably herein. The instructions can be stored in object code format for direct processing by a processor, or in any other computing device language including scripts or collections of independent source code modules that are interpreted on demand or compiled in advance. Functions, methods, and routines of the instructions are explained in more detail below.

Data 118 may be retrieved, stored, or modified by the one or more processors 112 in accordance with the instructions 116. For instance, although the subject matter described herein is not limited by any particular data structure, the data can be stored in computer registers, in a relational database as a table having many different fields and records, or XML documents. The data can also be formatted in any computing device-readable format such as, but not limited to, binary values, ASCII or Unicode. Moreover, the data can comprise any information sufficient to identify the relevant information, such as numbers, descriptive text, proprietary codes, pointers, references to data stored in other memories such as at other network locations, or information that is used by a function to calculate the relevant data.

The one or more processors 112 can be any conventional processors, such as a commercially available CPU. Alternatively, the processors can be dedicated components such as an application specific integrated circuit ("ASIC") or other hardware-based processor.

Although FIG. 1 functionally illustrates the processor, memory, and other elements of computing devices 110, 120, 130, and 140 as being within the same block, the processor, computer, computing device, or memory can comprise multiple processors, computers, computing devices, or memories that may or may not be stored within the same physical housing. For example, the memory can be a hard drive or other storage media located in housings different from that of the computing devices 110, 120, 130, and 140. Accordingly, references to a processor, computer, computing device, or memory will be understood to include references to a collection of processors, computers, computing devices, or memories that may or may not operate in parallel. For example, the computing device 110 may include server computing devices operating as a load-balanced server farm, distributed system, etc. Yet further, although some functions described below are indicated as taking place on a single computing device having a single processor, various aspects of the subject matter described herein can be implemented by a plurality of computing devices, for example, communicating information over network 160.

The one or more computing devices 110, 120, 130, and 140 can be at different nodes of a network 160 and capable of directly and indirectly communicating with other nodes of network 160. Although only a few computing devices are depicted in FIGs. 1 and 2, it should be appreciated that a typical system can include many connected computing devices, with each different computing device being at a different node of the network 160. The network 160 and intervening nodes described herein can be interconnected using various protocols and systems, such that the network can be part of the Internet, World Wide Web, specific intranets, wide area networks, or local networks. The network can utilize standard communications protocols, such as Ethernet, WiFi and HTTP, protocols that are proprietary to one or more companies, and various combinations of the foregoing. Although certain advantages are obtained when information is transmitted or received as noted above, other aspects of the subject matter described herein are not limited to any particular manner of transmission of information.

As an example, each of the computing devices 110, 120, 130, and 140 may include web servers capable of communicating with storage system 150 as well as the other computing devices via network 160. For example, one or more of server computing devices 110 may use network 160 to transmit and present information to a user, such as user 220, 230, or 240, on a display, such as displays 122, 132, or 142 of computing devices 120, 130, or 140. In this regard, computing devices 120, 130, and 140 may be considered client computing devices and may perform all or some of the features described herein.

Each of the client computing devices 120, 130, and 140 may be configured similarly to the server computing devices 110, with one or more processors, memory and instructions as described above. Each client computing device 120, 130, or 140 may be a personal computing device intended for use by a user 220, 230, 240, and have all of the components normally used in connection with a personal computing device such as a central processing unit (CPU), memory (e.g., RAM and internal hard drives) storing data and instructions, a display such as displays 122, 132, or 142 (e.g., a monitor having a screen, a touch-screen, a projector, a television, or other device that is operable to display information), and user input device 124 (e.g., a mouse, keyboard, or touch screen).

Although the client computing devices 120, 130, and 140 may each comprise a full-sized personal computing device, they may alternatively comprise mobile computing devices capable of wirelessly exchanging data with a server over a network such as the Internet. By way of example only, client computing device 120 may be a mobile phone or a device such as a wireless-enabled PDA, a tablet PC, or a netbook that is capable of obtaining information via the Internet.

As with memory 114, storage system 150 can be of any type of computerized storage capable of storing information accessible by the server computing devices 110, such as a hard-drive, memory card, ROM, RAM, DVD, CD-ROM, write-capable, and read-only memories. In addition, storage system 150 may include a distributed storage system where data is stored on a plurality of different storage devices which may be physically located at the same or different geographic locations. Storage system 150 may be connected to computing devices 110, 120, 130, and 140 via network 160 as shown in FIG. 1 and/or may be directly connected thereto (not shown).

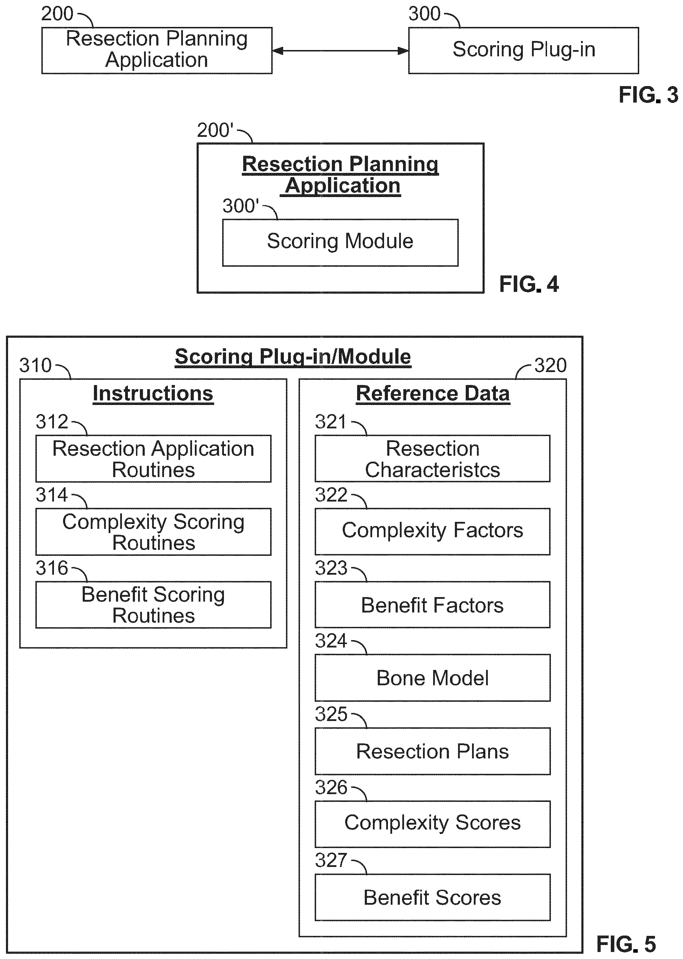

FIG. 3 depicts the general components that can be used in system 100 and that are configured to implement the methods described below. The first component is an existing Resection Planning Application 200. An exemplary resection planning application that can be utilized in system 100 can be found in U.S. Publication No. 2021/0282858. The second component is a Scoring Plug-In 300 that can communicate back and forth with Resection Planning Application 200 via an interface, such as a plugin API, for example.

FIG. 4 depicts an alternative configuration in which a Resection Planning Application 200' is modified or built from scratch to include an integrated Scoring Module 300'. In some embodiments, scoring Plug-In or Module 300/300' can be incorporated into commercially available orthopedic imaging software. However, in either embodiment, the Scoring Plugin or Module 300/300' is configured to operate autonomously in the background of the Resection Planning Application 200/200' to perform the methods described herein while a user uses the Resection Planning Application 200/200' to develop resection plans for a particular patient case. In other words, the Scoring Plug-In or Module 300/300' operates in real-time with limited user input or without user input while the user performs or can perform other functions in the Resection Planning Application 200.

FIG. 5 depicts the components of the Scoring Plug-In or Module 300/300', which includes instructions 310 and reference data 320. The instructions 310 include resection application routines 312, complexity scoring routines 314, and benefit scoring routines 316. The resection application routines 312 generally apply virtual resections to a virtual bone model, either automatically according to default settings or based on user defined parameters and performs all of the processing related to assessing the characteristics of such resections and resection plans so that such data can be used to score the benefit and complexity of a resection plan. Complexity and benefit scoring routines 314, 316 score the complexities and benefits, respectively, of each resection plan applied by the resection application routines 312.

Reference data 320 may be referred to during the implementation of the aforementioned routines. Such reference data 320 generally includes resection characteristics 321, complexity factors 322, benefit factors 323, a bone model 324, resection plans 325, complexity scores 326, and benefit scores 327. Resection characteristics 321 include various characteristics and parameters associated with particular types of bone resections as implemented in a virtual space, such as planar resections and hull-type resections, so that resection application routines 312 can use this data to apply one or more resection to bone model 324. The complexity and benefits factors 322, 323 include the various factors used by complexity and benefit scoring routines 314, 316 to score the benefits and complexities of a resection plan based on the particular resections of a resection plan 325 and their relationship relative to each other and the bone model. The complexity and benefit scores 326, 327 are the output of the complexity and benefit scoring routines 314, 316. Bone model 324 is a 3D virtual model of a patient's bone that may be obtained from a medical image.

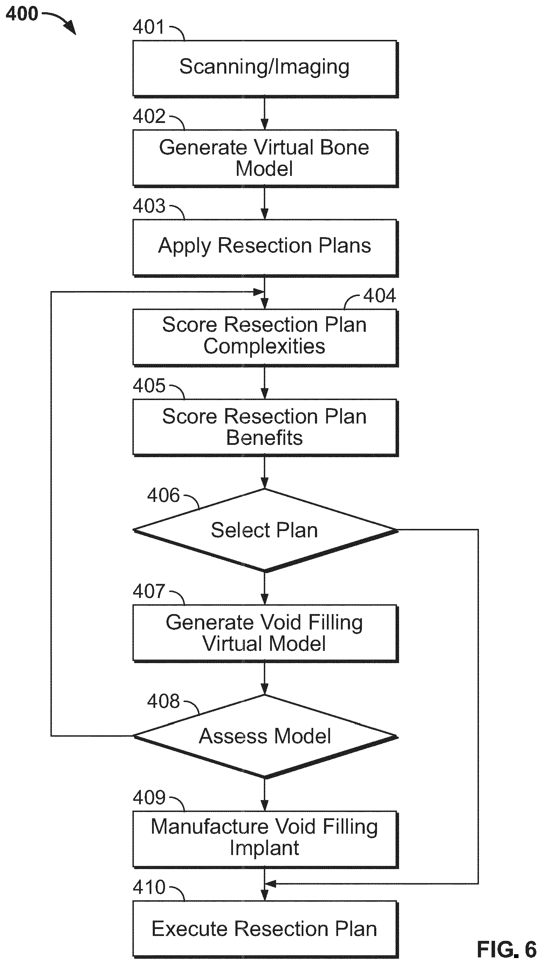

Flow diagram of FIG. 6 is an example method that may be performed by system 100. As shown in block 401 of this example method, a pre-operative medical image of a patient's bone is obtained. This is preferably performed using a CT scan. However, other medical imaging modalities may be utilized, such as MRI, PET, fluoroscopy, ultrasound or a hybrid such as PET/CT, for example. The data from the medical imaging is then stored in the memory of the system 100.

Once the data from the medical image has been obtained and loaded onto the memory accessible by the processor, a step 402 of generating a 3D virtual model of the bone from the medical image is performed. The processor may do this via segmentation in which the medical image is partitioned, and the pixels or voxels within each partition are analyzed by the processor to identify objects and their boundaries captured by the image. These objects and boundaries are then rendered into a corresponding 3D virtual model of the bone. Thus, through segmentation, the contours and features of the patient's bone are developed in a virtual space from the medical image. In cases where the bone has a tumor, the boundaries of the tumor may be delineated from healthy, non-diseased bone by the processor during the segmentation process via a thresholding technique or any other technique known in the art. The contours of the tumor are rendered in the 3D model allowing the surgeon to visualize the tumor's geometries and its boundaries relative to other anatomy, such as the articular surfaces of a femur or tibia, for example.

A step 403 of applying a plurality of resection plans to the virtual bone model in the virtual space is then performed. A resection plan is a pre-operative plan that specifies the parameters of each resection that is to be made to the bone so that the surgeon or robot following the plan removes the desired amount of bone at the desired locations. System 100 is configured so that each resection within a plan can be applied to the virtual bone model via a processor generated virtual control boundary that represents the resection. An exemplary application of such cutting boundary can be found in the U.S. Publication 2021/0282858. Each resection of a plan may be applied to the virtual bone model by the surgeon or automatically via processor. In this regard, system 100 may be configured to automatically generate a resection plan by automatically applying virtual boundaries representative of the resections to the virtual bone model based on default parameters or parameters set by the surgeon.

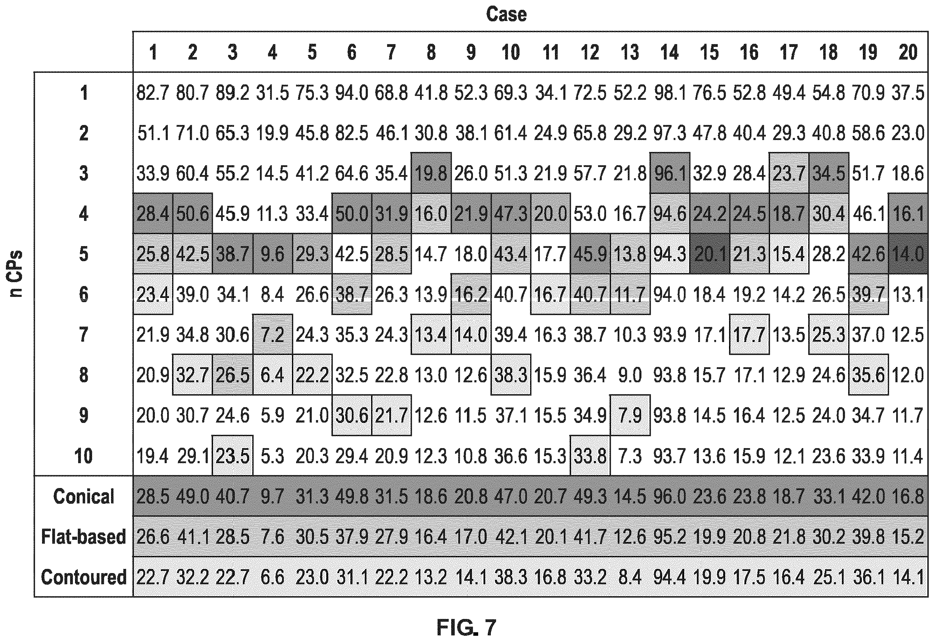

More than one resection plan can be alternatively used to achieve the same general objective. For example, an objective of a surgical procedure may be to remove a bone tumor. This can be done using one or more cut planes (CPs) to excise the tumor. As shown in the table of FIG. 7, for each case, 1 to 10 CPs may be implemented in a resection plan, or a more elaborate hull-type resection can be implemented. For example, a 1-CP resection plan generally involves cutting along a single plane through the bone which effectively amputates the end of the bone that contains the tumor. On the other hand, a 10-CP resection plan involves cutting along ten intersecting planes around the tumor. A hull-type resection is generally executed via a robot using a rotating burr and involves resecting around the tumor with a convex, curved geometry. Hull-type resections can be one of a conical hull, flat-based hull, or contoured hull resection. A conical hull resection forms a cone shape, a flat-based hull resection forms a frustoconical shape, and a contoured hull resection generally follows the contours of the tumor so that the resection is curvilinear in a plurality of two-dimensional views. The characteristics of each of these resections, such as characteristics 321, may be user defined or defined by the default settings of system 100 for application by the processor.

As shown in the table of FIG. 7, each of these resection plans removes a different volume of bone. For example, a 1-CP plan removes a much greater volume of bone than a 10-CP plan. Also, the hull-type resection plans each have a near equivalent planar resection plan in terms of the amount of bone removed. For example, in Case #1, a conical hull resection plan removes substantially the same amount of bone as a 4-CP resection plan, a flat-based hull resection plan removes substantially the same amount of bone as a 5-CP resection plan, and a contoured hull resection plan removes substantially the same amount of bone as a 6-CP resection plan.

If the primary motivation behind a resection plan were the minimization of bone waste, then a contoured hull resection plan or 10-CP resection plan would be implemented in every case. However, doing so would ignore other factors. System 100 takes these additional factors into account. Such general factors include, but are not limited to, biomechanics, implant fixation, and patient quality of life. System 100 incorporates these factors into a plan selection process by scoring the complexities and benefits of each plan so that the collective plans can be compared to each other and the optimal plan for the particular case can be selected, as discussed further below.

Thus, a plurality of resection plans are applied to the virtual bone model which, as mentioned above, may be performed in the virtual space by the surgeon or automatically by the processor. Any number of resection plans can be applied. However, it is preferable to apply ten separate planar resection plans (1-10 CPs), the hull-type resection plans, and optionally a manual resection plan so that they can each be scored for complexity and benefit and compared on those bases. A manual resection plan is a plan that is prepared by a surgeon based on experience, knowledge, and best practices and provides a baseline comparison value. In contrast, a CP plan is an algorithm generated resection plan that is autonomously prepared by the processor using current patient data, such as a segmented, 3D virtual bone and tumor model, for example.

The processor then performs steps 404 and 405 of scoring the complexities and benefits, respectively, for each resection plan. These steps may be performed after all of the resection plans have been generated either automatically or after being instructed by the user. Alternatively, steps 404 and 405 may be performed in the background of system 10 concurrently with step 403 of applying the resection plans to the virtual bone model.

In doing so, the processor may automatically associate a variety of complexity and benefit factors, such as factors 322 and 323, with each plan. In the example method described herein, these factors, which fit into the general categories mentioned above, include the number of cuts, the type of cut, the type of tooling that may be required to make the cut, the amount of bone removed by each cut, whether a cut preserves or sacrifices specified anatomy, and whether the cuts help constrain a void filling implant. As each resection plan is inputted into system 100, the processor may operate in the background of system 100 to identify the presence or absence of these factors and associate them with the specific resection plan. This data is then stored by the processor in the system's memory, such as in a relational database. The processor may also prompt the user, via a pop-up window or the like, to confirm assumptions made regarding a specific resection plan.

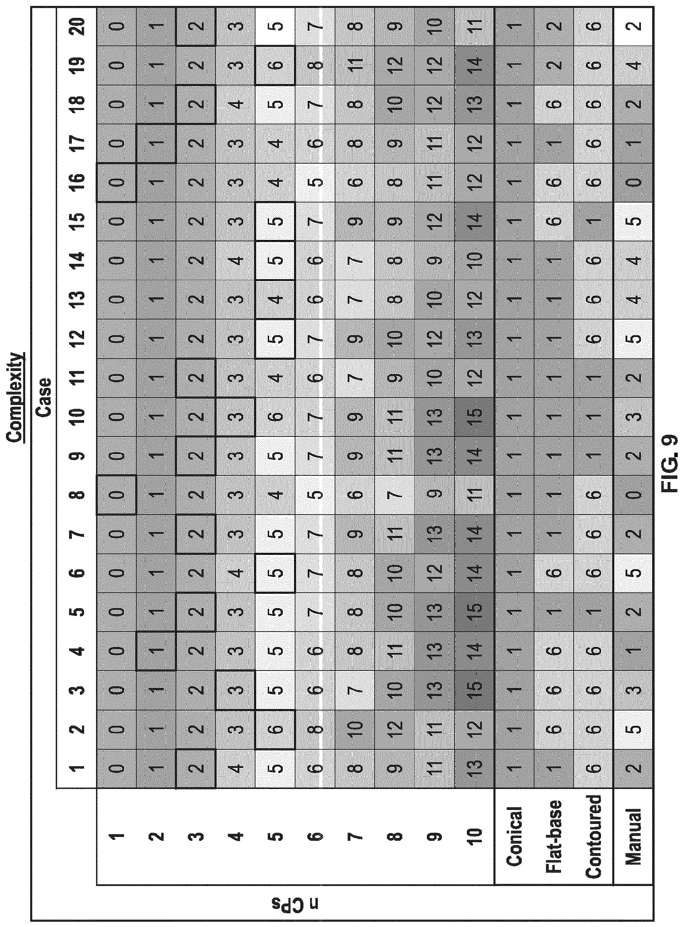

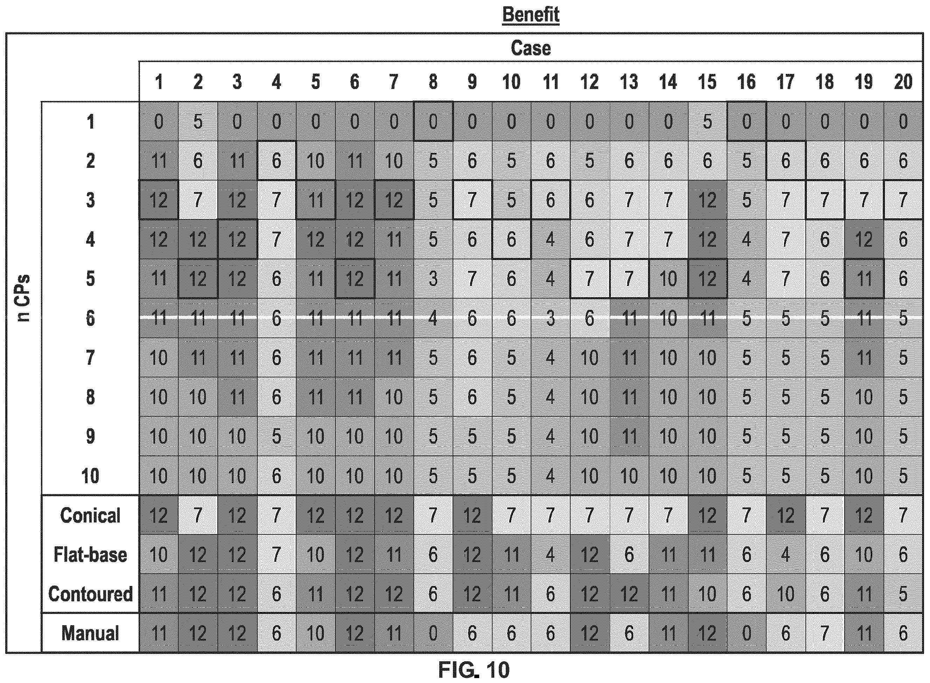

The processor subsequently or concurrently determines the complexity and benefit scores for each plan and then stores those scores in the database so that the scores are tabulated similar to that in the tables of FIGs. 9 and 10. As shown in these tables, complexity and benefit scores are tabulated for each proposed resection plan for each of 20 patient cases. Each patient case includes 1-10 CP plans, three hull-type plans (i.e., conical, flat-base, contoured), and a manual plan. However, it should be understood that this aggregation of resection plans is exemplary, and that other resection plans not mentioned, or, on the other hand, less than the mentioned plans can be analyzed for their relative complexities and benefits. The processor can display these tables to the user via the with color coding in the form of a heat map to visually illustrate the magnitude of the complexity and benefit scores for the totality of resection plans.

An exemplary framework for the complexity scoring step 404 will now be described. Such framework takes into consideration a plurality of complexity factors 322 which in this example include the presence of blind and/or fan cuts, the requirement of novel tooling to access one or more CPs, and the total number of CPs to be used for a particular resection plan. It should be understood that this list of factors is not exhaustive and should not be limiting but is instead an example of how system may assess the complexity and benefit of a defined resection plan. These factors and how they may be scored are now explained in greater detail.

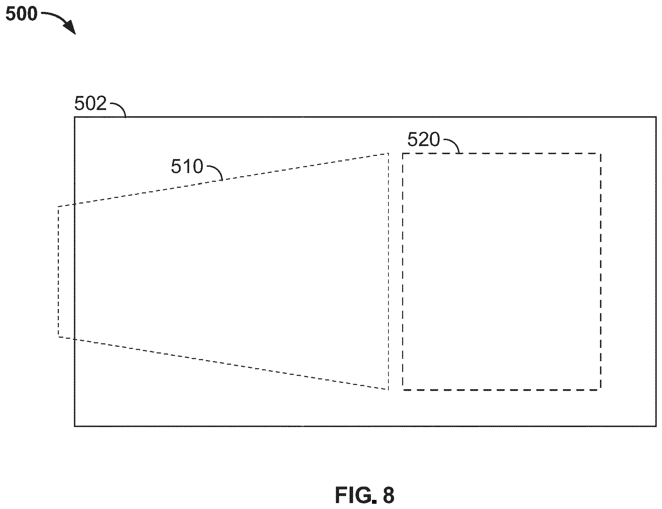

FIG. 8 is a schematic representation of a bone 500 which includes a fan cut 510 and a blind cut 520. Fan cut 510 is a cut that intersects a surface 520 of bone 500 but is narrowest at the entry point so that the CP increases in width the further it extends from bone surface 510. Blind cut 520 is a cut that does not intersect surface 502 of bone 500. The complexity score is increased by 1 point for each one of these cut-types present in a resection plan. For example, the presence of five fan cuts would increase the complexity score by 5 points.

Some cuts may require the use of novel orthopedic tools. Such tools may be designed for increased accessibility for performing elaborate cuts, which may include a fan or blind cut. The requirement of novel tooling to access at least one cut increases the complexity score by 5 points.

A resection plan generally increases in complexity for each additional cut added to the plan. Thus, for every additional CP beyond the first CP, the complexity score is increased by 1 point. For example, a complexity score would increase by 2 points for a resection plan that implements three CPs.

Hull resections are scored similarly to CP plans in that fan/blind cuts, special tooling, and number of cuts are considered in the complexity analysis. However, unlike with CP plans, hull resections are provided with a starting complexity score, which may be 1 point, for example. This contrasts with the manual resection plans which are given an initial complexity score of 0.

An exemplary framework for benefit scoring step 405 will now be described. Such framework takes into consideration a plurality of benefit factors 323 which include the preservation of articular anatomy (e.g., femoral or tibial condyles), the creation of innate and/or rotational fixation, and the reduction of bone waste. Again, this list of factors is not exhaustive but is instead an example of how system 100 may assess the benefits of a defined resection plan. These factors and how they may be scored are now explained in greater detail.

Bone cancers are commonly found in the metaphysis of long bones. For example, malignant tumors of the femur and tibia are often found near the knee joint. In this regard, a resection plan may sacrifice one or both condyles of the respective tibia and femur in order to remove the tumor, while another resection plan may spare one or both condyles. The benefit score is increased by 5 points for each condyle that is preserved. For example, if a malignant tumor is present in a distal femur, a resection plan that spares the lateral condyle increases the benefit score by 5 points, while a resection plan that spares both the lateral and medial condyles increases the benefit score by 10 points. The weighting of this factor relative to the other factors demonstrates and considers the significant value of being able to preserve the natural joint.

The presence of innate rotational and/or translation fixation refers to the ability of the bone after it is resected to constrain the implant rotationally and/or translationally. In other words, where two CPs meet within a bone, the line of intersection between those two CPs fixes the orientation of the reconstructive void filling implant thereby preventing the implant from rotating relative to the bone irrespective of the presence of mechanical fixation features, such as screws, flanges, and plates, for example. However, this intersection line does not necessarily fix the position of the void filling implant. Thus, the implant could translate relative to the intersecting planar surfaces. However, the presence of a third CP intersecting with one or both of the other CPs will restrict translation, as well as rotation, thereby providing a level of innate mechanical fixation between the void in bone and the matched implant surfaces. If the resection plan provides innate rotational and/or translational fixation to the reconstructive implant, the benefit score is increased by 1 point for each constraint. Thus, a resection plan that innately constrains a void filling implant both rotationally and translationally increases the benefit score by 2 points.

As previously discussed, it is beneficial to reduce bone waste. However, rather than reward each additional CP added to a resection plan, a resection plan is instead penalized if the next additional CP fails to substantially reduce bone waste. In this regard, the default bone waste benefit score for any resection plan is zero. Any additional CP which reduces bone waste by greater than 20% of the previous resection plan that is absent that CP receives no penalty, and the score remains at zero. For example, no penalty is applied where a fourth CP of a 4-CP plan reduces bone waste by greater than 20% beyond that of a 3-CP plan. On the other hand, each additional CP which reduces bone waste by 10% to 20% of the previous resection plan, the benefit score is decreased by 1 point. Additionally, the benefit score is decreased by 2 points for each additional CP that reduces bone waste by 10% or less of the previous resection plan.

Again, hull-type resections are provided a starting benefit score, such as 1 point, for example, and scored similarly to CP plans in that condylar preservation, innate fixation, and bone waste reduction are considered in the benefit analysis. This contrasts with the manual resection plans which are given an initial benefit score of 0.

It should be understood that the weightings assigned to the complexity and benefit factors described above are exemplary. A surgeon may override the default settings of system 10 and apply additional factors and/or assign different weights.

### Examples

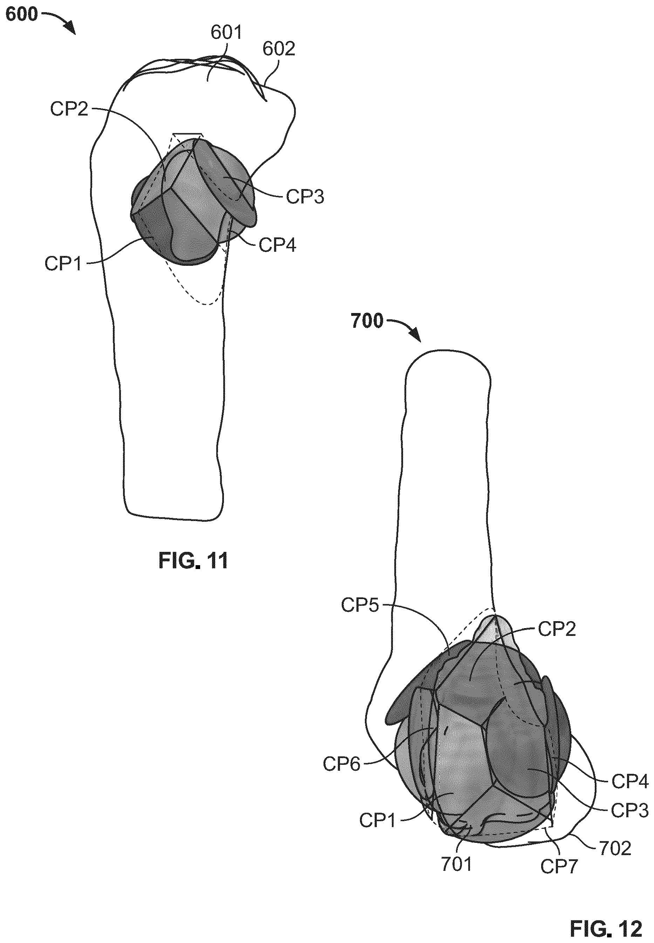

Examples of the complexity and benefit scoring step are now described with reference to the tables of FIGs. 9 and 10 and the bone models of FIGs. 11 to 14.

### Example #1: Case 3

FIG. 11 illustrates a virtual bone model of a tibia 600 for Case #3. The exemplary resection plan applied to this virtual bone model includes four CPs to remove a malignant tumor from tibia 600. Regarding the complexity score, there are no fan cuts or blind cuts present, and no special tooling is needed. The plan includes four total CPs which is three more CPs than a 1-CP plan. Therefore, the total complexity score is 3.

Regarding the benefit score, the four CPs intersect to provide both innate rotational and translational fixation to a reconstruction implant configured to fill the void formed by the resections which adds 2 points to the benefit score. Also, both tibial condyles 601, 602 are preserved which adds 10 points to the benefit score. CP4 saves at least 20% or more bone waste than the previous resection plan (*i.e.,* the 3-CP plan). Thus, there is no penalty to the benefit score for the addition of CP4. The total benefit score is therefore 12. These complexity and benefits scores are tabulated are shown in the tables of FIGs. 9 and 10, respectively.

### Example #2: Case 9

FIG. 12 illustrates a virtual bone model of a femur 700 for Case #9. The exemplary resection plan applied to this virtual bone model includes 7 CPs to remove a malignant tumor from femur 700. Regarding the complexity score, three CPs result in either fan cuts or blind cuts. For example, CP1, CP2, and CP3 are fan cuts. This increases the complexity score by 3 points. No special tooling is required to perform these cuts. The plan includes seven total CPs which is six more than a 1-CP plan. Therefore, the total complexity score is 9.

Regarding the benefit score, the CPs intersect to provide rotational fixation, but not translational fixation which increases the benefit score by 1 point. CP7 also saves at least 20% bone waste than the previous resection plan (i.e., the 6-CP plan) so that there is no penalty to the benefit score. The first condyle 701 is sacrificed while the second condyle 702 is preserved thereby increasing the benefit score by 5 points. Therefore, the total benefit score for this plan is 6.

### Example #3: Case 13

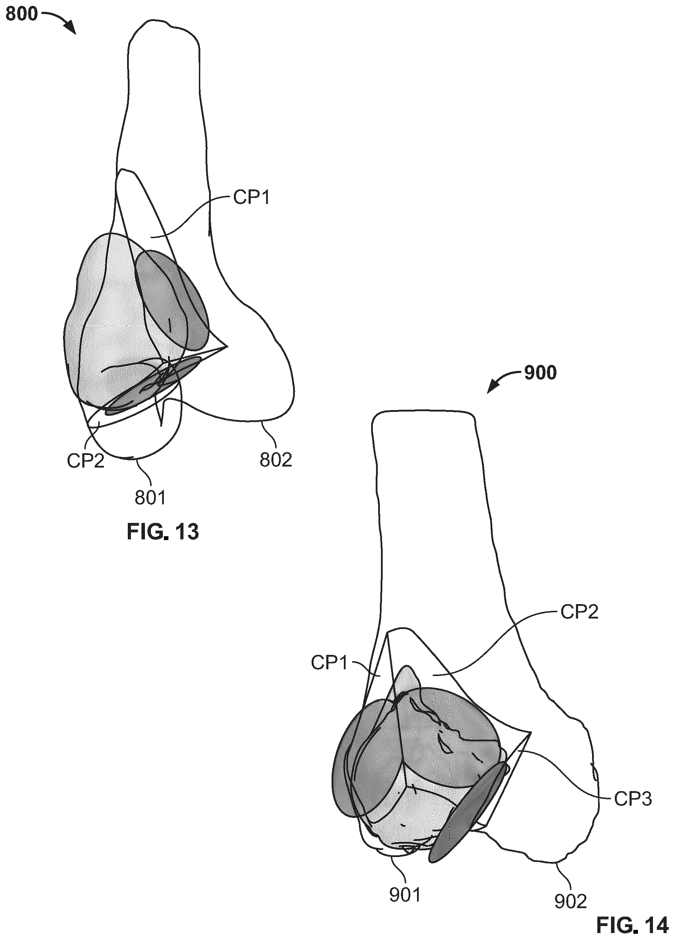

FIG. 13 illustrates a virtual bone model of a femur 800 for Case #13. The exemplary resection plan applied to this virtual bone model includes two CPs to remove a malignant tumor from the femur 800. Regarding the complexity score, there are no fan cuts or blind cuts, and no special tooling is needed. The plan includes two total CPs which is one more CP than a 1-CP plan. Therefore, the total complexity score is 1.

Regarding the benefit score, the CP1 and CP2 intersect to provide innate rotational fixation, but not translation fixation for a void filling implant. This increases the benefit score by 1 point. CP2 also saves at least 20% bone waste than the previous resection plan (i.e., the 1-CP plan). Therefore, there is no penalty for the inclusion of CP2 to the resection plan. However, CP2 partially intersects the first condyle 801 resulting in its removal. Nonetheless, CP2 spares the second condyle 802 thereby increasing the benefit score by 5 points so that the total benefit score of the illustrated resection plan is 6.

### Example #4: Case 18

FIG. 14 illustrates a virtual bone model of a femur 900 for Case #18. The exemplary resection plan applied to this virtual bone model includes three CPs to remove a malignant tumor from femur 900. Regarding the complexity score, there are no fan cuts or blind cuts, and no special tooling is required. The plan includes three total CPs which is two more CPs than a 1-CP plan. Therefore, the total complexity score is 2.

Regarding the benefit score, the CP1, CP2, and CP3 intersect to provide innate rotational fixation and translation fixation for a void filling implant. This increases the benefit score by 2 points. CP3 also saves at least 20% bone waste beyond that of a previous resection plan (i.e., the 2-CP plan). Therefore, there is no penalty for the addition of CP3 to the resection plan. However, the plan results in the removal of the first condyle 901 while the second condyle 902 is preserved thereby increasing the benefit score by 5 points. Therefore, the total benefit score is 7.

Other intraoperative considerations can be evaluated for their complexities and benefits. For example, a procedure may be performed robotically which may come with its own unique complexities and benefits (e.g., complexities and benefits of path and registration, reduction in cutting time (relative to a manual procedure), required soft tissue access, required repositioning of the patient, expected visibility of trackers, and the like). Such scores may be scored as intraoperative considerations in addition to the resection plan complexities and benefits discussed above.

Another factor that may be considered when evaluating a resection plan's complexity and benefit is the extent of potential bone landmark variation for an expected registration error. Robotics and patient specific guides can generally achieve high precision and have been shown to increase the accuracy of resections as compared to those made freehand or using navigation alone. However, high accuracy is more difficult to guarantee as, regardless of the assistive technology utilized, some degree of error and uncertainty remains in the execution of the surgical plan. Cut accuracy using assistive technologies largely depends on the accuracy of the patient-to-image registration process. However, in practice, there is always some amount of registration error present, the practical effect of which is a mismatch between where the robotic system has identified the target bone as being located and the bone's real position and orientation in space. The inventors have found that, through a Monte-Carlo simulation approach, bone landmarks may have a normally distributed range of variability in terms of absolute distance from its theoretical registration position over a number of registration error simulations. In other words, a designated bone landmark has a theoretical registration position according to a preoperative plan and an actual registration position according to the actual execution of the preoperative plan. The larger the deviation of the actual registration position from the theoretical registration position, the less accuracy of the resection and implant placement can be expected.

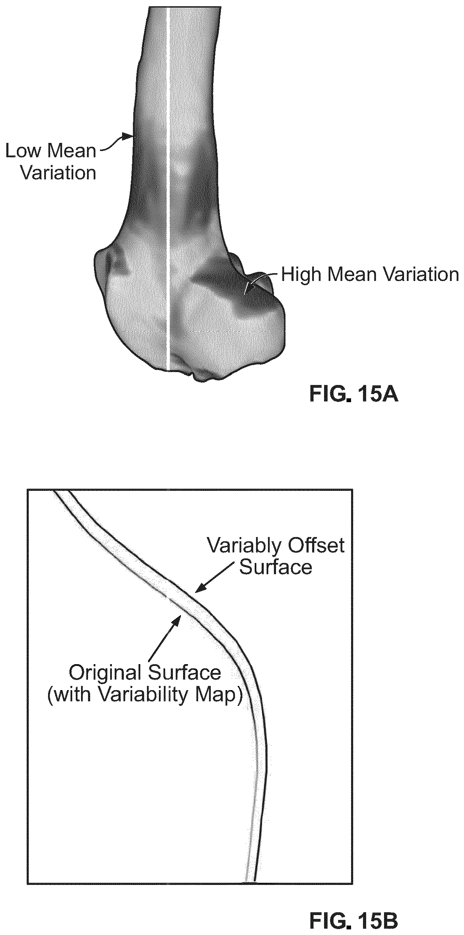

As shown in FIGs. 15A and 15B, the processor executing the Monte Carlo approach can generate a heat map illustrating the magnitude of potential landmark variation across the entire surface of a bone. More specifically, FIG. 15A is a heat map depicting an expected effect of registration error over a bone (here a distal femur) at the specified landmarks where cold regions are those in which registration errors have a small (low mean variation) and hot regions have a large (high mean variation) overall variation effect. FIG. 15B depicts an offset of a portion of the surface of FIG. 15A from its theoretical registration position (original surface) to its expected position (variably offset surface) based on an expected registration error distribution. As can be seen with reference to these figures, the potential variation of bone landmarks for a registration error may be greater in regions of highly contoured geometries. It is therefore preferable to avoid placing an implant in such highly contoured regions of potentially high variation in order to help mitigate negative consequences of registration error, such as impingement, for example. In this regard, a resection plan's benefit and/or complexity score can take into account whether or not the resection and final implant placement occurs at regions of high potential variability. Where the resection and implant placement avoids such areas, the complexity score is not enhanced, whereas the benefits score is increased. Conversely, the complexity score increases where a resection in a resection plan intersects a high mean variation region, and the benefit score is not enhanced. The magnitude of increase of such complexity and/or benefit score can be binary based on whether or not intersection of a high variation region occurs. Alternatively, the magnitude of increase of the complexity and/or benefit score can be a function of how much of the overall resection involves a high variation region, such as a percentage of the total exterior surface area of the bone resected.

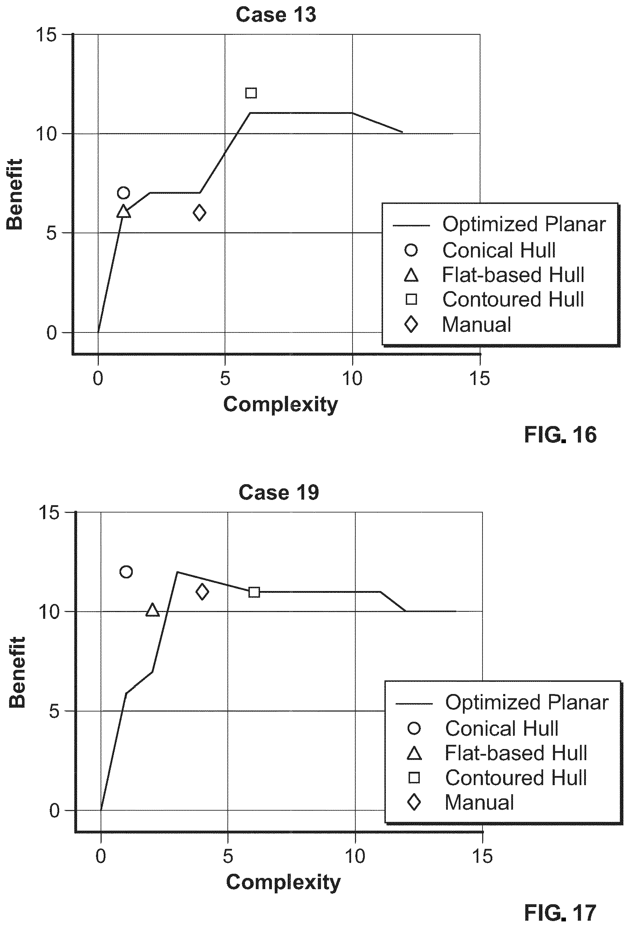

Once the resection complexity and benefit scores are determined, a step of selecting a resection plan 406 from the plurality of resection plans is performed. To aid the selection, the processor may generate a graph in two-dimensional Cartesian coordinate system which an X-axis represents the complexity scores, and a Y-axis represents the benefit scores. FIG. 16 is an exemplary graph from Case #13, and FIG. 17 is an exemplary graph from Case #19, as tabulated in the tables of FIGs. 9 and 10. As can be observed from the FIGs. 15 and 16, the further up the vertical axis a resection plan is located, the greater the benefit, while the further to the right on the horizontal axis the resection plan is located, the greater its intraoperative complexity. The relative position of points gives an indication of whether a resection plan is excessively complex for the benefit it provides, or conversely, the benefit it provides is worth the additional complexity. The line representing all of the planar optimized resection plans provides a baseline to help determine which resection plan should be selected. For example, icons representing the complexity and benefits of the hull-type and manually prepared resection plans are plotted relative to this line. If an icon is below or to the right of the line, then that particular plan offers less benefit and is more complex than a corresponding planar optimized resection plan and likely will not be considered for selection. Conversely, if an icon is above and/or to the left of the line, then that resection plan is either more beneficial for the same complexity or offers equivalent benefit for less complexity.

Each of these graphs also demonstrate that at some point, adding more complexity to the procedure provides very little, if any, additional benefit. These graphs help determine where that transition takes place. Indeed, system 100 may have a default setting in which it will automatically identify a preferred plan or couple of plans that are located at a transition in the line's slope from positive trending upward in benefit to zero trending toward more complex with no added benefit. For example, in case 13, the 3-CP planar resection plan and the 6-CP planar resection plan may be identified by system 100 as preferred plans as these plans are located at a slope transition to zero. System 100 may also be configured so that the hull-type or manual resection plans that are positioned to the left and above the line are automatically designated as preferred plans, while those to the right and below the line are excluded as non-preferred. The surgeon may also override the default preferred plan selection criteria and instead apply their own criteria to system 100 for automated preferred plan identification.

Once a resection plan is selected, a step of generating a virtual void filling implant model 407 may be performed. In this regard, the virtual bone model with the selected resection plan applied to it may be exported to a CAD application as a digital twin. Such CAD application may be an existing CAD application, such as Solidworks of Dassault Systèms SE (Vèlizy-Villcoublay France) and AutoCad of Autodesk (San Rafael, CA), for example. From there, a virtual implant model may be generated from the negative space created by the resection plan. This may be done automatically by the processor of system 100 using the boundaries set by the selected resection plan and an extension of bone's exterior surface. The virtual implant model therefore is provided with a conformal geometry that matches the resection boundaries.

Once the virtual implant model is generated, a step of assessing the virtual implant model 408 may be performed. For example, the virtual implant model can be analyzed using finite element analysis (FEA) to evaluate stress concentrations, fatigue resistance, and the ultimate strength of the implant, build orientation, lattice configuration, and fixation options. for example. Thus, the virtual implant model can be exported from the CAD application to an FEA application, or the CAD application may have an FEA module associated with it. The FEA application may be an existing FEA application, such as ANSYS of Ansys (Canonsburg, PA).

Other physical characteristics of the virtual implant can be evaluated for complexity and/or benefit. For example, the number of screws and/or flanges and their relative orientations and positions needed for fixation of the implant. Other considerations may include manufacturing characteristics like total build time, cleaning complexity, what post-processing is needed and its complexity, overall cost, and engineering safety factors.

Further evaluation of the virtual implant model may include evaluating complexity and benefit as implemented in a surgical procedure. For example, the ease of implantation and the degree of initial fixation provided by the implant can be evaluated in the virtual space through an implantation simulation. Furthermore, once the virtual implant is implanted in a virtual bone model, kinematic simulations may also be performed in the virtual space to assess range of motion through a patient gait cycle or any anatomic anomalies that might affect performance.

Thus, the characteristics of the virtual implant model can be scored for its contribution to the complexity and benefit of the pre-operative/treatment plan. Such scores may be fed back into the Resection Planning Application 200/200' to revise the initial complexity and benefit scores for the resection plan to output a treatment plan score and to determine if another resection plan should be selected or further evaluated. In some embodiments, rather than selecting a single resection plan and generating an implant model from this plan, virtual implant models can be generated for each resection plan (or more than one resection plan) and then be collectively assessed so that the revised complexity and benefit scores can be compared on an apples-to-apples basis. In even further embodiments, complexity/benefit scores associated with the virtual implant model may remain separate from the resection plan complexity/benefit scores. In other words, such implant complexity/benefit scores may not be used to revise the resection plan complexity/benefit scores so that the surgeon can evaluate each score individually. Keeping these scores separate, rather than aggregating them into a total complexity/benefit treatment plan score, may help provide the surgeon transparency as to each individual score so that the surgeon can make a more informed decision based on each aspect of the treatment protocol. Thus, scoring of each aspect of a treatment plan can be output to the surgeon in the form of a single graph associated with each aspect, such as those in FIGs. 15 and 16. Alternatively, the relative complexity/benefit scores for each aspect can be integrated into a respective treatment score (e.g., treatment complexity score and treatment benefit score).

Once a resection plan is finally selected and the virtual implant model is generated, a step of manufacturing the void filling implant 409 is performed. This can be done using any applicable manufacturing technique, including additive manufacturing. Where additive manufacturing is to be used, the implant model may be transferred to an additive manufacturing application which uses the implant model to print the physical implant. Thus, the void filling implant may be formed layer-by-layer using an additive layer manufacturing (ALM), *i.e.,* 3D printing, process so no separate connection mechanism is necessary to bring together any of the features of such implant. In some examples, ALM processes are powder-bed based and involve one or more of selective laser sintering (SLS), selective laser melting (SLM), and electron beam melting (EBM), as disclosed in U.S. Pat. Nos. 7,537,664; 8,728,387; 9,180,010; and 9,456,901 as well as U.S. Patent Publication No. 2006/0147332. Other methods of ALM, which can be used to form the herein described implants, include stereolithography (SLA), fused deposition modeling (FDM), and continuous liquid interface production (CLIP).

The step of executing the resection plan 410 may then be performed. The plan can be executed entirely manually by the surgeon, robotically, or by the surgeon with a robotically assisted haptic feedback system. An exemplary robotic-assisted system that can be utilized is disclosed in U.S. Patent No. 10,716,630. If the procedure is performed robotically or robotically assisted, then the virtual bone model with the selected resection plan overlayed may be exported to the robotic system which uses the boundaries of the resection plan to perform the desired cuts. Once the desired cuts are performed, the void filling implant is implanted in the void. As shown in FIG. 6, the step of executing the resection plan 410 can be performed after selecting the plan 406 without performing steps 407, 408, and 410.

It should be understood that other aspects of a treatment plan can be evaluated for complexity and benefits. Such aspects may include robotic execution of a surgical procedure

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A computer-implemented method of selecting a bone resection plan for implementation in a surgical procedure, the method comprising:
generating, by one or more processors, a virtual bone model from a medical image taken of a bone of a mammalian subject; and
applying, by the one or more processors executing a resection planning application, a plurality of resection plans (325) to the virtual bone model, each of the plurality of resection plans (325) having at least one virtual control boundary representing a resection of specified geometry and defining a region of bone to be removed,
the method being **characterized in that**
each of the plurality of resection plans has at least one complexity factor (322) and at least one benefit factor (323) associated therewith, the at least one complexity factor (322) and the at least one benefit factor (323) being determined by the one or more processors in the background of the resection planning application upon application of each of the resection plans (325) to the virtual bone model; and **in that**
the method further comprises:
determining, by the one or more processors, a complexity score (326) for each of the resection plans based on the at least one complexity factor (322), wherein the complexity score (326) is determined in the background of the resection planning application;
determining, by the one or more processors, a benefit score (327) for each of the resection plans based on the at least one benefit factor (323), wherein the benefit score (327) is determined in the background of the resection planning application; and
selecting an optimal resection plan from the plurality of resection plans (325) based on the complexity and benefit scores (326, 327) of the plurality of resection plans (325) for execution in the surgical procedure.

2. The method of claim 1, wherein a first series of the resection plans (325) applied to the virtual bone model each comprise one or more resections of planar geometry such that a first resection plan includes one cut plane and each subsequent resection plan in the series has one more cut plane than the previous cut plan.

3. The method of claim 2, wherein a second series of the resection plans (325) applied to the virtual bone model each comprise one of a conical hull resection, flat-base hull resection, and conformal hull resection.

4. The method of claim 2, wherein the complexity factors (322) include at least one of the total number of cut planes in the resection plan, the need for novel tooling, and whether one of the resection in a resection plan is a blind cut or a fan cut.

5. The method of claim 4, wherein the benefit factors (323) include at least one of the presence of innate and/or translational fixation, the preservation of an articular surface, and the amount of bone waste reduced relative to a resection plan with one less cut plane.

6. The method of claim 1, wherein the selecting step includes comparing the complexity and benefit scores (326, 327) of the plurality of resection plans (325).

7. The method of claim 6, wherein comparing the complexity and benefit scores (326, 327) includes plotting, by the one or more processors, the complexity and benefit scores (326, 327) on a line in a two-dimensional Cartesian coordinate system and selecting, by the one or more processors, a resection plan at a transition location between a positive slope and a zero slope of the line.

## Patentansprüche

1. Computerimplementiertes Verfahren zum Auswählen eines Knochenresektionsplans zur Durchführung eines chirurgischen Eingriffs, wobei das Verfahren Folgendes umfasst:
Erzeugen eines virtuellen Knochenmodells aus einem medizinischen Bild, das von einem Knochen eines Säugetiers aufgenommen wurde, durch einen oder mehrere Prozessoren; und
Anwenden einer Vielzahl von Resektionspläne (325) auf das virtuelle Knochenmodell durch den einen oder die mehreren Prozessoren, die eine Resektionsplanungsanwendung ausführen, wobei jeder der Vielzahl von Resektionsplänen (325) mindestens eine virtuelle Kontrollgrenze aufweist, die eine Resektion einer bestimmten Geometrie darstellt und einen zu entfernenden Knochenbereich definiert,
wobei das Verfahren **dadurch gekennzeichnet ist, dass**
jeder der Vielzahl von Resektionsplänen mindestens einen Komplexitätsfaktor (322) und mindestens einen damit verbundenen Nutzenfaktor (323) aufweist, wobei der mindestens eine Komplexitätsfaktor (322) und der mindestens eine Nutzenfaktor (323) von dem einen oder den mehreren Prozessoren im Hintergrund der Resektionsplanungsanwendung bei Anwendung jedes der Resektionspläne (325) auf das virtuelle Knochenmodell bestimmt werden; und dass
das Verfahren ferner Folgendes umfasst:
Bestimmen eines Komplexitätswerts (326) für jeden der Resektionspläne basierend auf dem mindestens einen Komplexitätsfaktor (322) durch den einen oder die mehreren Prozessoren, wobei der Komplexitätswert (326) im Hintergrund der Resektionsplanungsanwendung bestimmt wird;
Bestimmen eines Nutzenwerts (327) für jeden der Resektionspläne basierend auf dem mindestens einen Nutzenfaktor (323) durch der einen oder die mehreren Prozessoren, wobei der Nutzenwert (327) im Hintergrund der Resektionsplanungsanwendung bestimmt wird; und
Auswählen eines optimalen Resektionsplans aus der Vielzahl von Resektionsplänen (325) basierend auf dem Komplexitäts- und dem Nutzenwert (326, 327) der Vielzahl von Resektionsplänen (325) zur Ausführung beim chirurgischen Eingriff.

2. Verfahren nach Anspruch 1, wobei eine erste Reihe der auf das virtuelle Knochenmodell angewendeten Resektionspläne (325) jeweils eine oder mehrere Resektionen mit planarer Geometrie umfasst, so dass ein erster Resektionsplan eine Schnittebene enthält und jeder nachfolgende Resektionsplan in der Reihe eine Schnittebene mehr als der vorherige Schnittplan aufweist.

3. Verfahren nach Anspruch 2, wobei eine zweite Reihe der auf das virtuelle Knochenmodell angewendeten Resektionspläne (325) jeweils eine von einer konischen Hüllresektion, einer flachbasierten Hüllresektion und einer konforme Hüllresektion umfasst.

4. Verfahren nach Anspruch 2, wobei die Komplexitätsfaktoren (322) mindestens eines von der Gesamtanzahl der Schnittebenen im Resektionsplan, der Notwendigkeit neuer Werkzeuge und der Frage, ob einer der Schnitte in einem Resektionsplan ein Blindschnitt oder ein Fächerschnitt ist, umfassen.

5. Verfahren nach Anspruch 4, wobei die Nutzenfaktoren (323) mindestens eines von dem Vorhandensein von angeborener und/oder translationaler Fixierung, der Erhaltung einer Gelenkfläche und der Menge an Knochenabfall, die im Vergleich zu einem Resektionsplan mit einer Schnittebene weniger reduziert wird, umfassen.

6. Verfahren nach Anspruch 1, wobei der Auswahlschritt das Vergleichen der Komplexitäts- und Nutzenwerte (326, 327) der Vielzahl von Resektionsplänen (325) umfasst.

7. Verfahren nach Anspruch 6, wobei das Vergleichen der Komplexitäts- und Nutzenwerte (326, 327) das Aufzeichnen, durch den einen oder die mehreren Prozessoren, der Komplexitäts- und Nutzenwerte (326, 327) auf einer Linie in einem zweidimensionalen kartesischen Koordinatensystem und das Auswählen, durch den einen oder die mehreren Prozessoren, eines Resektionsplans an einer Übergangsstelle zwischen einer positiven Steigung und einer Nullsteigung der Linie umfasst.

## Revendications

1. Procédé mis en œuvre par ordinateur pour sélectionner un plan de résection osseuse à mettre en œuvre dans une procédure chirurgicale, le procédé comprenant :
génération, par un ou plusieurs processeurs, d'un modèle d'os virtuel à partir d'une image médicale prise d'un os d'un sujet mammifère ; et
application, par le ou les processeurs exécutant une application de planification de résection, d'une pluralité de plans de résection (325) au modèle d'os virtuel, chacun de la pluralité de plans de résection (325) comportant au moins une limite de contrôle virtuelle représentant une résection de géométrie spécifiée et définissant une région osseuse à retirer,
le procédé étant **caractérisé en ce que**
chacun de la pluralité de plans de résection comporte au moins un facteur de complexité (322) et au moins un facteur d'avantage (323) qui lui sont associés, le au moins un facteur de complexité (322) et le au moins un facteur d'avantage (323) étant déterminés par le ou les processeurs en arrière-plan de l'application de planification de résection lors de l'application de chacun des plans de résection (325) au modèle d'os virtuel ; et **en ce que**
le procédé comprend en outre :
détermination, par le ou les processeurs, d'un score de complexité (326) pour chacun des plans de résection sur la base de l'au moins un facteur de complexité (322), le score de complexité (326) étant déterminé en arrière-plan de l'application de planification de résection ;
détermination, par le ou les processeurs, d'un score d'avantage (327) pour chacun des plans de résection sur la base de l'au moins un facteur d'avantage (323), le score d'avantage (327) étant déterminé en arrière-plan de l'application de planification de résection ; et
sélection d'un plan de résection optimal parmi la pluralité de plans de résection (325) sur la base des scores de complexité et d'avantage (326, 327) de la pluralité de plans de résection (325) pour exécution dans la procédure chirurgicale.

2. Procédé selon la revendication 1, dans lequel une première série de plans de résection (325) appliqués au modèle d'os virtuel comprend chacun une ou plusieurs résections de géométrie plane de telle sorte qu'un premier plan de résection comprend un plan de coupe et que chaque plan de résection suivant dans la série comporte un plan de coupe de plus que le plan de coupe précédent.

3. Procédé selon la revendication 2, dans lequel une deuxième série de plans de résection (325) appliqués au modèle d'os virtuel comprend chacun une résection conique, une résection à base plate et une résection conforme.

4. Procédé selon la revendication 2, dans lequel les facteurs de complexité (322) comprennent au moins l'un parmi le nombre total de plans de coupe dans le plan de résection, la nécessité d'un outillage nouveau et le fait que l'une des résections dans un plan de résection soit une coupe aveugle ou une coupe en éventail.

5. Procédé selon la revendication 4, dans lequel les facteurs d'avantage (323) comprennent au moins l'un parmi la présence d'une fixation innée et/ou translationnelle, la préservation d'une surface articulaire et la quantité de déchets osseux réduite par rapport à un plan de résection avec un plan de coupe en moins.

6. Procédé selon la revendication 1, dans lequel l'étape de sélection comprend une comparaison des scores de complexité et d'avantage (326, 327) de la pluralité de plans de résection (325).

7. Procédé selon la revendication 6, dans lequel la comparaison des scores de complexité et d'avantage (326, 327) comprend un tracé, par le ou les processeurs, des scores de complexité et d'avantage (326, 327) sur une ligne dans un système de coordonnées cartésiennes bidimensionnel et une sélection, par le ou les processeurs, d'un plan de résection à un emplacement de transition entre une pente positive et une pente nulle de la ligne.
